# EUROPÄISCHE PATENTSCHRIFT

(11) **EP 0 897 910 B1**
(45) Veröffentlichungstag und Bekanntmachung des Hinweises auf die Patenterteilung: **06.11.2002**
(21) Anmeldenummer: 98114790.3
(22) Anmeldetag: 06.08.1998
(51) Int. Cl.: C07C 309/58

(54) **Verfahren zur Herstellung von 2-Carboxyy-5-nitro-benzolsulfonsäure und deren Salzen durch Oxidation**
Process for the preparation of 2-carboxy-5-nitrobenzene sulphonic acid and its salts by oxidation
Procédé pour la préparation de l'acide 2-carboxy-5-nitrobenzène sulphonique et de ses sels par oxydation

(30) Priorität: 19.08.1997 DE 19735879
(43) Veröffentlichungstag der Anmeldung: 24.02.1999
(73) Patentinhaber: Bayer CropScience GmbH, 65929 Frankfurt/Main (DE)
(72) Erfinder: Ford, James Mark Dr., 65815 Bad Soden (DE)

(56) Entgegenhaltungen:
- DATABASE WPI Section Ch, Week 8304 Derwent Publications Ltd., London, GB; Class B05, AN 83-08062K XP002084797 & JP 57 200353 A (NIPPON KAYAKU KK) , 8. Dezember 1982 & CHEMICAL ABSTRACTS, vol. 99, no. 5, 1. August 1983 Columbus, Ohio, US; abstract no. 38208f, Seite 500;

## Beschreibung

Die Erfindung betrifft das technische Gebiet der chemischen Verfahren zur Herstellung von Zwischenprodukten, die zur Herstellung von Wirkstoffen, anderen Zwischenprodukten oder zur Anwendung bestimmten Produkten auf unterschiedlichen Gebieten, z.B. auf den Gebieten Pharma, Pflanzenschutz, Polymere und/oder Farbstoffe, eingesetzt werden können.

In US-A-4,608,205, DE-A-4,334,949, DE-A-4,236,902 und JP-A-57,036,121 (vgl. Chem. Abstracts 97:92991(1998)) und Handbüchem der Chemie ist die Verbindung 2-Carboxy-5-nitrobenzolsulfonsäure als Zwischenprodukt für die Synthese unterschiedlichster Wirkstoffe beschrieben. Die Herstellung des Zwischenprodukts kann dabei z.B. durch Oxidation von 2-Methyl-5-nitrobenzolsulfonsäure erfolgen, wobei speziell die Reaktionen mit erhitzter basischer Kaliumpermanganatlösung (siehe US-A-4,608,205) mit Salpetersäure bei 130-220°C (siehe US-A-2,675,405), mit Luftsauerstoff in Gegenwart von Mangan- und/oder Kobaltsalzen (siehe US-A-5,175,351, CS-A-149,998, CS 150,000) oder mit Hypochlorit in Gegenwart von Schwermetallperoxiden und Alkalihydroxiden (siehe JP-A-57,200,353, Derwent Abstracts 83-08062K (1998)) erwähnt seien. Die genannten Oxidationsverfahren sind mit Nachteilen verbunden, die besonders stark bei der Übertragung auf größere Ansätze z.B. Ansätze im industriellen Maßstab auftreten.

Neben den Verarbeitungs- und Abfallproblemen, die mit der Handhabung großer Mengen an KMnO₄ verbunden sind, treten bei dieser basischen Oxidation von 2-Methyl-5-nitrobenzol-sulfonsäure stark ausbeutemindemde Nebenreaktionen wie die gut dokumentierte, rasche Dimerisation der Ausgangsverbindung auf; siehe Chem. Ber. 1886 (19) 3234, Chem. Berichte 1897 (30) 3236 und J. Am. Chem. Soc. 1954 (76) 2725.
Die Oxidation mit Salpetersäure, welche die baseninduzierte Dimerisation vermeidet, ist andererseits bei den erforderlichen hohen Reaktionstemperaturen derart exotherm, daß ihre Durchführung im industriellen Maßstab erheblicher Kontrolle bedarf und besondere Maßnahmen zur Minimierung des Risikos getroffen werden müssen.

Die schwach-basischen Oxidationen mit Hypochlorit (JP-57200353) oder Luft (US-A-5175351) benötigen jedoch Schwermetalle als Katalysatoren und sind wegen des damit verbundenen Aufwands für Aufarbeitung und Abfallbeseitigung ebenfalls nachteilig. Außerdem können dabei Peroxide auftreten, welche ein Explosionsrisiko darstellen. Ein Explosionsrisiko bedeutet auch der Einsatz von organischen Lösungsmitteln im industriellen Maßstab bei den genannten Luftoxidationen.

Aus US-A-5,565,608 ist bereits bekannt, daß man die isomere 5-Methyl-2-nitrobenzolsulfonsäure im Labormaßstab gut zur isomeren 5-Carboxy-2-nitrobenzolsulfonsäure oxidieren kann, wenn man die Oxidation mit NaOCI (Natriumhypochlorit) bei 50 - 150°C, vorzugsweise 100 - 110°C ohne Schwermetalloxide durchführt. Überraschenderweise gelingt diese Oxidation bei analoger Anwendung der dort beschriebene Bedingungen auf die 2-Methyl-5-nitrobenzolsulfonsäure nicht. Bereits bei Temperaturen (z.B. 45 - 50°C) unterhalb der für die Oxidation erforderlichen Reaktionstemperaturen tritt starke Gasentwicklung auf, so daß die Reaktion in Reaktionskesseln von ökonomisch vertretbarer Größe nicht durchgeführt werden kann.

Bei höheren Temperaturen (z.B. Rückflußtemperatur) wird die Gasentwicklung zwar verringert, jedoch ist die Oxidation des Natrium- oder Kaliumsalzes der 2-Methyl-5-nitrobenzolsulfonsäure mit NaOCl unter Rückflußbedingungen wesentlich langsamer als die Zersetzung des Reagenzes. So ergibt die Umsetzung mit 6 Äquivalenten wäßriger Natriumhypochloritlösung (13,5 Gew.-% NaOCl) nur eine 45 %ige Umsetzung zur gewünschten Carbonsäure. Wie bereits erwähnt ist außerdem bekannt, daß die Oxidation mit basischer Natriumhypochlorit-Lösung das Dimere 4,4'-Dinitrobibenzyl-2,2'-disulfonsäure oder 4,4'-Dinitrostilben-2,2'-disulfonsäure als Nebenprodukte ergibt (siehe DE-A-115410, DE-A-106961 und Chem. Ber. 1897 (30) 3097).

Weitere Oxidationen von methylsubstituierten Aromaten unter Verwendung von Natriumhypochlorit sind bekannt (siehe US-A-3,790,624 und WO-9427959), bei denen jedoch Schwermetalle als Katalysatoren eingesetzt werden.

Eine Aufgabe der Erfindung wird daher in der Bereitstellung eines Verfahrens gesehen, mit dem 2-Methyl-5-nitrobenzolsulfonsäure ohne die oben genannten Nachteile im industriellen Maßstab oxidiert werden kann.

Gegenstand der Erfindung ist ein Verfahren zur Herstellung der Verbindung der Formel (I) oder deren Salze 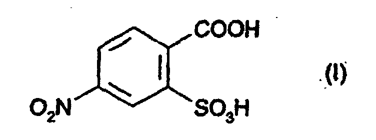 dadurch gekennzeichnet, daß man eine Verbindung der Formel (II) oder deren Salze 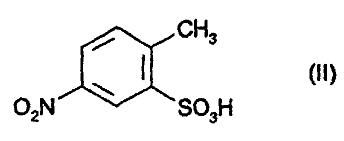 mit einem stöichiometrischen Äquivalent oder einem Überschuß eines Metallhypochlorits, vorzugsweise LiOCl, NaOCI, KOCI oder Ca(OCl)₂, in Gegenwart einer Metallbase aus der Gruppe der Alkalimetallhydroxide oder Alkalimetallcarbonate oder Gemischen davon ohne den Zusatz von Schwermetallsalzen oxidiert.

Das stöchiometrische Äquivalent für die Oxidation der Methylgruppe in der Verbindung der Formel (II) sind 3 Mol Hypochlorit-Anion oder 3 Mol eines einwertigen Hypochlorits pro Mol Verbindung der Formel (II).

Als Metallbasen sind die Alkalimetallhydroxide LiOH, NaOH und KOH sowie die Alkalimetallcarbonate Na₂CO₃ und K₂CO₃ und deren Mischungen besonders geeignet.

Die Verbindung der Formel (I) wird als freie Carboxybenzolsulfonsäure, als Monosalz, Disalz oder eine Mischung der Verbindungen erhalten, abhängig vom pH-Wert bei der Aufarbeitung. Aus ökonomischen Gründen sind Alkalimetall- und Erdalkalimetallsalze bevorzugt, insbesondere Alkalimetallsalze wie Natrium- und Kaliumsalze, und wird wegen vereinfachter Reaktionführung Wasser als Lösungsmittel eingesetzt.

In der Regel kann das Verfahren so durchgeführt werden, daß man zunächst eine Mischung aus Verbindung (II) oder deren Salz, Wasser und dem Hypochlorit herstellt, beispielsweise durch Zugabe von 2-Methyl-5-nitrobenzolsulfonsäure zu mindestens einem Äquivalent eines Alkalimetallhydroxids und/oder Alkalimetallcarbonats (d.h. mindestens 1 Moläquivalent Base pro Mol Verbindung (II)), das vorher mit einem stöchiometrischen Äquivalent oder mit einem Überschuß bezogen auf Verbindung (II) einer wäßrigen Lösung oder Suspension des Hypochlorits vermischt worden ist.

Alternativ kann die 2-Methyl-5-nitrobenzolsulfonsäure auch zu einer wäßrigen Mischung des Hypochlorits, Hydroxids und gegebenenfalls Carbonats gegeben werden, welche durch£inleiten von mindestens einem stöchiometrischen Äquivalent Chlorgas, vorzugsweise bei Temperaturen von 5°C oder weniger als 5°C, in einen Überschuß von wäßrigem Metallhydroxid und/oder Metallcarbonat erhalten wurde. Überschüssiges Hypochlorit kann nach der Oxidation aus dem Ansatz wiedergewonnen und dem nächsten Ansatz zugeführt werden.

Die erhaltene wäßrige Mischung, enthaltend Verbindung (II), Hypochlorit, Hydroxid und/oder Carbonat, läßt man dann unter Erwärmen reagieren, beispielsweise unter Erwärmen auf 60°C oder darüber, vorzugsweise auf 60 °C bis zum Rückfluß (ca. 110°C). Die Oxidationsreaktion kann durch portionsweise Zugabe weiterer Base, vorzugsweise weiterem wäßrigem Alkalimetallhydroxid, kontrolliert werden. Wenn die Reaktion durch Zugabe weiterer Base kontrolliert wird, hängt die optimale Geschwindigkeit der Zugabe von der Ansatzgröße und der Konzentration der Reaktionspartner ab. Vorzugsweise wird eine Zugabegeschwindigkeit eingestellt, die einen pH-Wert in der Reaktionsmischung von pH 10 oder mehr als pH 10, beispielsweise pH 10 bis 11 gewährleistet.

Bei einer Reaktionstemperatur von 60 bis 75°C ist die Zugabe von Hydroxid oder Carbonat in der Regel geschwindigkeitsbestimmend für den Ablauf der Reaktion, so daß eine zusätzliche pH-Bestimmung entbehrlich sein kann.

Bei höheren Reaktionstemperaturen, z.B. auch Rückflußtemperatur, wird vorzugsweise ständig ein Überschuß von Alkalimetallhydroxid bzw. Alkalimetallcarbonat eingestellt und dadurch erreicht, daß die Gesamtmenge an Base, z.B. 1 bis 3 Moläquivalente bereits am Anfang der Reaktion vorhanden ist.

Alternativ kann 2-Methyl-5-nitrobenzolsulfonsäure oder ein Salz davon als Feststoff, Lösung oder Suspension in Wasser über einen Zeitraum von mehreren Stunden zu einer Mischung des Hypochlorits mit dem Hydroxid und/oder dem Carbonat unter Einhaltung der genannten Bedingungen für den pH-Wert und gegebenenfalls Zugabe weiterer Base zugegeben werden.

Das Hypochlorit wird mit einem stöchiometrischen Äquivalent oder im Überschuß, vorzugsweise in einer Gesamtmenge von 3 bis 10 Mol, insbesondere 4 bis 7 Moläquivalent Hypochlorit pro Mol Verbindung der Formel (II) eingesetzt; die Gesamtmenge Hypochlorit kann beispielsweise zusammen bereits am Anfang der Reaktion eingesetzt werden oder ergibt sich aus der Summe der portionsweise oder stetig zugegebenen Anteile über die gesamte Reaktionszeit, wobei vorzugsweise ständig Hypochlorit im Überschuß zur Verbindung der Formel (II) vorliegt. Der Überschuß an Hypochlorit kann dabei auch durch Einleiten von Chlorgas in die alkalische Reaktionslösung erreicht werden.

Das Oxidationsverfahren läßt sich aus technologischer Sicht einfach durchführen und liefert das Produkt (I) in hervorragenden Ausbeuten und Reinheiten. Bei der Durchführung tritt keine Gasentwicklung oder Zersetzung des Hypochlorits auf, wie sie bei der analogen Anwendung der Verfahrensbedingungen nach US-A-5,565,608 (= ohne Basenzusatz) auf die Oxidation der Verbindung der Formel (II) beobachtet wird.

Die Aufarbeitung des Rohprodukts kann nach üblichen Methoden der Laboratoriumspraxis oder Verfahrenstechnik erfolgen. Das Produkt der Formel (I) liegt nach der Oxidation zunächst als Monosalz oder Disalz vor, vorzugsweise als Dinatrium- oder Dikaliumsalz, welches direkt durch Filtration, gegebenenfalls nach Aussalzen, isoliert werden kann.

Alternativ kann das Produkt nach Ansäuem mit einer Säure, vorzugsweise einer Mineralsäure wie Salzsäure oder Schwefelsäure, als Monosalz, vorzugsweise wegen der geringen Löslichkeit als Kaliumsalz oder Natriumsalz oder einer Mischung davon oder auch als freie Säure, in Abhängigkeit vom pH-Wert, der eingestellt wird, erhalten werden.

Kristallisierung und Rückgewinnung des Produkts als Mono- oder Disalz kann durch Zugabe eines wasserlöslichen Salzes eines Alkalimetall- oder Erdalkalimetallhydroxids erleichtert werden.

Vorteilhaft kann ein Teil des überschüssigen Oxidationsmittels als Hypochlorit zurückgewonnen und wiederverwendet werden. Dazu wird z.B entweder vor oder nach Isolierung des Produkts das Reaktionsmedium angesäuert und das entweichende Chlorgas aus überschüssigem Hypochlorit in eine alkalische Lösung eingeleitet, z.B. in wäßriges Alkalimetallhydroxid oder eine Lösung aus Alkalimetallhydroxid und wäßrigem Alkalimetallhypochlorit, die gegebenenfalls Ausgangsstoff oder Produkt der Oxidation enthält.

Gegenstand der Erfindung sind auch die Verwendung der erfindungsgemäß hergestellten Verbindungen der Formel (I) zur Herstellung von Weiterverarbeitungsprodukten, vorzugsweise auf den anfangs genannten technischen Gebieten. Gegenstand sind auch die Verfahren zur Herstellung von Weiterverarbeitungsprodukten der Verbindungen (I), vorzugsweise solchen mit einer Teilstruktur der Formel (IIIa) oder (IIIb), 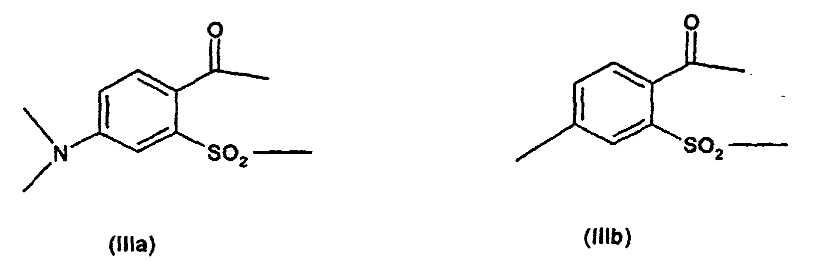 worin die freien Bindungen die Bindungsstellen der Teilstruktur darstellen (nicht zu verwechseln mit der teilweise üblichen Kurzschreibweise für Methylgruppen), dadurch gekennzeichnet, daß in einem Verfahrensschritt das weiter oben erläuterte erfindungsgemäßen Verfahren zur Herstellung der Verbindungen (I) durchgeführt wird.

In den folgenden Beispielen beziehen sich Prozentangaben auf das Gewicht, sofern nichts anderes definiert ist.

### Beispiele

### Variante A

1 kg 79,8 %ige 2-Methyl-5-nitirobenzolsulfonsäure (3,67 mol) wurden zu einer Mischung aus 371 ml 40 %iger wäßrigem Kaliumhydroxid (3,70 mol) und 10,5 l (22,35 mol) wäßrigen Natriumhypochlorit gegeben. Die erhaltene Suspension wurde auf 65°C erhitzt und bei dieser Temperatur mit 768 ml 40 %igem wäßrigem Kaliumhydroxid (7,67 mol) mit der Zugabegeschwindigkeit von 1,07 ml/min versetzt. Nach vollständiger Zugabe wurde noch 4 Stunden bei 65°C gerührt und danach 1,4 kg (18,78 mol) Kaliumchlorid zugegeben. Nach Abkühlen auf 50 - 60°C säuerte man tropfenweise mit konzentrierter Salzsäure (37 %ig, 920 ml, 11,1 mol) über 1 bis 2 Stunden bei 50 - 60°C an, wobei das dabei gebildete Chlorgas in überschüssigem wäßrigen Kaliumhydroxid abgefangen wurde. Nach Abkühlen auf 15 - 20°C unter Durchleiten von Stickstoffgas, wobei restliches Chlorgas entfernt wurde und weiterem Rühren von 30 bis 60 min bei 15 - 20°C wurde das Produkt 2-Carboxy-5-nitrobenzolsulfonsäure-mono-kaliumsalz durch Filtration isoliert und bei 75°C/100 mbar getrocknet. Man erhielt das Produkt als weißes Pulver; Ausbeute: 1101 g; 86,7 %iges Produkt (91,3 % der Theorie).

### Variante B

27,2 g 79,8 %ige 2-Methyl-5-nitrobenzolsulfonsäure (0,1 mol) wurden zu einer Mischung aus 10,1 ml 40 %igem wäßrigem Kaliumhydroxid (0,1 mol) und 141 ml (0,3 mol) wäßrigem Natriumhypochlorit gegeben. Die erhaltene Suspension wurde auf 65°C erhitzt und bei dieser Temperatur mit 23 ml 40 %igem wäßrigem Kaliumhydroxid (0,23 mol) mit der Zugabegeschwindigkeit von 0,04 ml/min versetzt. Nach vollständiger Zugabe wurde auf 0°C gekühlt und dann mit 12,24 g (0,3 mol) KOH versetzt. Während 30 min wurden dann 10,64 g (0,15 mol) Chlorgas bei dieser Temperatur eingeleitet. Die Temperatur wurde danach auf 65°C erhöht und wäßriges KOH (40 %ig, 9,0 ml, 0,9 mol) bei 0,04 ml/min zugeführt und anschließend noch 2 Stunden bei 65°C gerührt. Nach Abkühlen auf 50°C säuerte man tropfenweise mit konzentrierter Salzsäure (37 %ig, 18 ml, 0,22 mol) über 1 Stunde bei 50°C an, kühlte auf 15 - 20°C unter Durchleiten von Stickstoffgas, wobei restliches Chlorgas entfernt wurde, und rührte noch 2 Stunden bei 15 - 20°C. Das Produkt 2-Carboxy-5-nitrobenzolsufonsäure-mono-kaliumsalz erhielt man nach Filtration und Trocknen bei 75°C/100 mbar als weißes Pulver; Ausbeute: 31 g; 76,5 %iges Produkt (83,1 % der Theorie).

### Variante C

2-Methyl-5-nitrobenzolsulfonsäure (79,8 %ig, 40,8 g, 0,15 mol) wurde zu einer Mischung aus 32 ml 40 %igem wäßrigen KOH (0,32 mol), 40,8 g (0,3 mol) K₂CO₃ und 420 ml (0,71 mol) wäßrigem NaOCl gegeben. Unter raschem Rühren wurde die erhaltene Suspension auf 85°C erhitzt und 30 min bei 85°C gehalten, 30 min auf 90°C erhitzt und dann 3,5 Stunden auf Rückfluß (103-104°C) erhitzt.
Die Reaktionsmischung kühlte man dann auf 15-20°C ab und rührte noch 30 min bei dieser Temperatur.
Nach Filtration und Trocknen bei 75°C/100 mbar erhielt man ein cremefarbenes Pulver in einer Ausbeute von 42,8 g 94,9 %iges 2-Carboxy-5-nitrobenzolsulfonsäure-mononatriummonokalium-disalz (88,2 % der Theorie).

## Patentansprüche

1. Verfahren zur Herstellung der Verbindung der Formel (I) oder deren Salze, 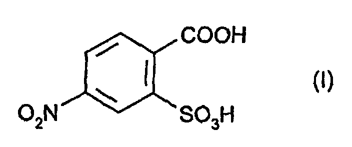 **dadurch gekennzeichnet, daß** man eine Verbindung der Formel (II) oder deren Salze, 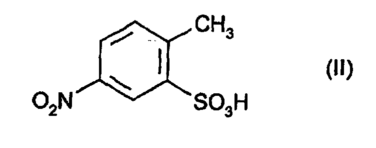 mit einem stöchiometrischen Äquivalent oder einem Überschuß eines Metallhypochlorits in Gegenwart einer Metallbase aus der Gruppe der Alkalimetallhydroxide oder Alkalimetallcarbonate oder Gemischen davon ohne den Zusatz von Schwermetallsalzen oxidiert.

2. Verfahren nach Anspruch 1, **dadurch gekennzeichnet, daß** ein Metallhypochlorit aus der Gruppe LiOCl, NaOCl, KOCl und Ca(OCl)₂ sowie Mischungen davon eingesetzt wird.

3. Verfahren nach Anspruch 1 oder 2, **dadurch gekennzeichnet, daß** ein Alkalimetallhydroxid aus der Gruppe LiOH, NaOH und KOH und Mischungen davon eingesetzt wird.

4. Verfahren nach einem der Ansprüche 1 bis 3, **dadurch gekennzeichnet, daß** ein Alkalimetallcarbonat aus der Gruppe Na₂CO₃ und K₂CO₃ und Mischungen davon eingesetzt wird.

5. Verfahren nach einem der Ansprüche 1 bis 4, **dadurch gekennzeichnet, daß** es in Gegenwart von Wasser erfolgt.

6. Verfahren nach einem der Ansprüche 1 bis 5, **dadurch gekennzeichnet, daß** die Oxidationsreaktion bei 60 bis 110°C durchgeführt wird.

7. Verfahren nach einem der Ansprüche 1 bis 6, **dadurch gekennzeichnet, daß** ein Überschuß an Metallhypochlorit durch Einleiten von Chlorgas in die alkalische Reaktionslösung hergestellt oder eingehalten wird.

8. Verfahren nach einem der Ansprüche 1 bis 7, **dadurch gekennzeichnet, daß** 3 bis 10 Moläquivalent Hypochlorit pro Mol Verbindung der Formel (II) eingesetzt wird.

9. Verfahren nach einem der Ansprüche 1 bis 8, **dadurch gekennzeichnet, daß** überschüssiges Hypochlorit oder ein Teil davon aus der Reaktionsmischung nach der Oxidation zurückgewonnen wird.

10. Verfahren nach einem der Ansprüche 1 bis 9, **dadurch gekennzeichnet, daß** es unter Ausschluß von Schwermetallsalzen durchgeführt wird.

## Claims

1. A process for preparing the compound of the formula (I) or salts thereof, 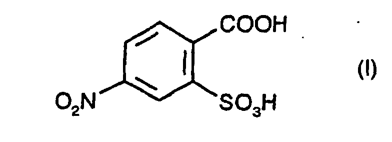 which comprises oxidizing a compound of the formula (II) or salts thereof 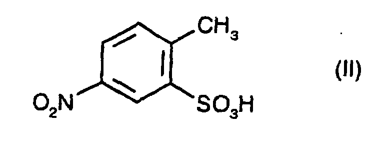 with a stoichiometric equivalent or an excess of a metal hypochlorite in the presence of a metal base from the group of the alkali metal hydroxides or alkali metal carbonates or mixtures thereof, without addition of heavy metal salts.

2. The process as claimed in claim 1, wherein a metal hypochlorite from the group consisting of LiOCl, NaOCl, KOCl and Ca(OCl)₂ and mixtures thereof is used.

3. The process as claimed in claim 1 or 2, wherein an alkali metal hydroxide from the group consisting of LiOH, NaOH and KOH and mixtures thereof is used.

4. The process as claimed in any of claims 1 to 3, wherein an alkali metal carbonate from the group consisting of Na₂CO₃ and K₂CO₃ and mixtures thereof is used.

5. The process as claimed in any of claims 1 to 4, wherein the process is carried out in the presence of water.

6. The process as claimed in any of claims 1 to 5, wherein the oxidation reaction is carried out at from 60 to 110°C.

7. The process as claimed in any of claims 1 to 6, wherein an excess of metal hypochlorite is prepared or maintained by introducing chlorine gas into the alkaline reaction solution.

8. The process as claimed in any of claims 1 to 7, wherein from 3 to 10 molar equivalents of hypochlorite are employed per mole of compound of the formula (II).

9. The process as claimed in any of claims 1 to 8, wherein excess hypochlorite or a part thereof is recovered from the reaction mixture after the oxidation.

10. The process as claimed in any of claims 1 to 9, wherein the process is carried out with exclusion of heavy metal salts.

## Revendications

1. Procédé pour la préparation du composé de formule (I) ou de ses sels, 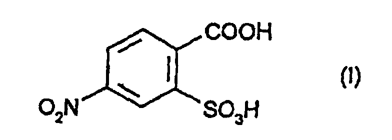 **caractérisé en ce qu'**on oxyde un composé de formule (II) ou ses sels, 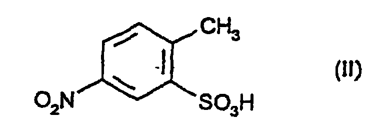 avec un équivalent stoechiométrique ou un excès d'un hypochlorite de métal en présence d'une base d'un métal choisie parmi les hydroxydes de métaux alcalins ou les carbonates de métaux alcalins ou leurs mélanges sans. l'addition de sels de métaux lourds.

2. Procédé selon la revendication 1, **caractérisé en ce qu'**on utilise un hypochlorite de métal choisi parmi LiOCl, NaOCl, KOCl et Ca(OCl)₂ ainsi que leurs mélanges.

3. Procédé selon la revendication 1 ou 2, **caractérisé en ce qu'**on utilise un hydroxyde de métal alcalin choisi parmi LiOH, NaOH et KOH et leurs mélanges.

4. Procédé selon l'une des revendications 1 à 3, **caractérisé en ce qu'**on utilise un carbonate de métal alcalin choisi parmi Na₂CO₃ et K₂CO₃ et leurs mélanges.

5. Procédé selon l'une des revendications 1 à 4, **caractérisé en ce qu'**il est réalisé en présence d'eau.

6. Procédé selon l'une des revendications 1 à 5, **caractérisé en ce que** la réaction d'oxydation est réalisée entre 60 et 110°C.

7. Procédé selon l'une des revendications 1 à 6, **caractérisé en ce qu'**on produit et on respecte un excès d'hypochlorite de métal par introduction de chlore gazeux dans la solution de réaction alcaline.

8. Procédé selon l'une des revendications 1 à 7, **caractérisé en ce qu'**on utilise de 3 à 10 équivalents molaires d'hypochlorite par mol de composé de formule (II).

9. Procédé selon l'une des revendications 1 à 9, **caractérisé en ce qu'**on récupère du mélange réactionnel après l'oxydation 1'hypochlorite en excès ou une partie de celui-ci.

10. Procédé selon l'une des revendications 1 à 9, **caractérisé en ce qu'**il est réalisé en excluant les sels de métaux lourds.
